## Europäisches Patentamt

## European Patent Office

## Office européen des brevets

(19)

(11) Veröffentlichungsnummer: **0 048 679**
**B1**

(12) **EUROPÄISCHE PATENTSCHRIFT**

(45) Veröffentlichungstag der Patentschrift:
**27.06.84**

(51) Int. Cl.³: **C 07 C  87/60, C 07 C  85/26**

(21) Anmeldenummer: **81710044.9**

(22) Anmeldetag: **12.09.81**

(54) **Verfahren zur Herstellung von 3,3'-Dichlor-4,4'-diamino-biphenyl-dihydrochlorid.**

(30) Priorität: **18.09.80  DE 3035221**

(43) Veröffentlichungstag der Anmeldung:
**31.03.82 Patentblatt 82/13**

(45) Bekanntmachung des Hinweises auf die Patenterteilung:
**27.06.84 Patentblatt 84/26**

(84) Benannte Vertragsstaaten:
**DE FR GB IT**

(56) Entgegenhaltungen:
**DE - C - 547 986**
**US - A - 2 839 578**

(73) Patentinhaber: **HOECHST AKTIENGESELLSCHAFT,**
**Postfach 80 03 20, D-6230 Frankfurt am Main 80 (DE)**

(72) Erfinder: **Habig, Kurt, Dr., Hundertmorgenring 98,**
**D-6082 Mörfelden-Walldorf (DE)**
Erfinder: **Baessler, Konrad, Dr., Drosselweg 1,**
**D-6230 Frankfurt am Main 80 (DE)**

# Beschreibung

3,3'-Dichlor-4,4'-diamino-biphenyl ist ein wichtiges Zwischenprodukt zur Herstellung von Azofarbmitteln. Für diesen Einsatzzweck, insbesondere als Bisdiazokomponente, wird es bevorzugt in Form des Dihydrochlorids eingesetzt, das jedoch, insbesondere bei der Herstellung von Pigmenten, bestimmte Reinheitsanforderungen erfüllen muß.

3,3'-Dichlor-4,4'-diamino-biphenyl wird üblicherweise aus o-Chlor-nitrobenzol hergestellt, das zum 2,2'-Dichlor-hydrazobenzol reduziert wird, das seinerseits der »Benzidinumlagerung« mit Mineralsäuren, beispielsweise Schwefelsäure, unterworfen wird.

Der Erfindung lag die Aufgabe zugrunde, reines 3,3'-Dichlor-4,4'-diamino-biphenyl-dihydrochlorid aus der schwefelsauren, wäßrigen Lösung des Sulfats durch Fällen mit einem Chloridionen abgebenden Mittel, Isolieren und Waschen des Fällungsprodukts mit einer wäßrigen, ein Chloridionen abgebendes Mittel enthaltenden Lösung herzustellen.

Ein solches Verfahren ist bereits aus der US-PS 29 96 546 bekannt. Bei diesem bekannten Verfahren wird nach der Umlagerung das Reaktionsgemisch erhitzt, bis das Sulfat in Lösung geht, das als Lösemittel dienende Toluol entfernt, die wäßrige, schwefelsaure Lösung des Sulfats mit Wasser verdünnt und aus dieser Lösung mit Natriumchlorid in einer Stufe das Hydrochlorid ausgefällt. Das abgetrennte Produkt wird dann zunächst mit einer wäßrigen, salzsauren Lösung eines polaren organischen Lösemittels, insbesondere Isopropanol, und anschließend mit einer salzsauren Kochsalzlösung gewaschen.

Dieses Verfahren hat den Nachteil, daß für das Waschen organische Lösemittel erforderlich sind, und weiterhin, daß eine aufwendige Aufarbeitung der Abwässer erforderlich ist.

Darüber hinaus wird ein Produkt erhalten, das nach der Isolierung mindestens 20% Mutterlauge enthält.

Erfindungsgemäß wird demgegenüber die annähernd gesättigte wäßrige Lösung des Sulfats auf eine Temperatur im Bereich von über 100°C bis zur Rückflußtemperatur erhitzt, hierbei etwa 50 bis etwa 70% der stöchiometrisch erforderlichen Menge des Fällungsmittels zugesetzt, anschließend auf eine Temperatur im Bereich von etwa 90 bis unter 100°C abgekühlt, weiteres Fällungsmittel zugegeben und auf etwa Zimmertemperatur abgekühlt.

Bevorzugte Ausgestaltungen der Erfindung werden im folgenden näher erläutert:

Aufgrund der Löslichkeit des Sulfats geht man zweckmäßig von einer etwa 85 bis etwa 100°C heißen Lösung aus, die vorteilhaft mit Hilfe geeigneter Klärhilfsmittel wie Aktivkohle zur Beseitigung der teerartigen Nebenprodukte geklärt wurde. Dieser Lösung kann man etwa 50% des Chloridionen abgebenden Fällungsmittels zusetzen, ohne daß es zu einer Ausfällung des Hydrochlorids kommt. Zweckmäßig wird die Lösung jedoch auf Temperaturen über 100°C erhitzt und bis zu etwa 65% der stöchiometrisch erforderlicher Menge an Fällungsmittel zugesetzt, wobei bei etwa 110°C im offenen Gefäß die Lösung siedet.

Als Chloridionen abgebendes Fällungsmittel kann ein geeignetes Chlorid, beispielsweise ein Alkalichlorid wie Natriumchlorid, dienen, vorzugsweise jedoch Salzsäure, da hierdurch die Aufarbeitung des Reaktionsmediums erleichtert wird.

Beim anschließenden gleichmäßigen und langsamen Abkühlen beginnt die Kristallisation des Hydrochlorids ab etwa 107°C, wobei sich größere Kristalle im Bereich von 100 bis 95°C bilden. Zur Vervollständigung der Kristallisation werden im Temperaturbereich von etwa 90 bis unter 100°C, bevorzugt von 95 bis 98°C weitere Mengen an Fällungsmittel zugesetzt. Vorteilhaft gibt man zunächst nur die restliche stöchiometrisch erforderliche Menge an Fällungsmittel oder einen geringen Überschuß zu und nach einer gewissen Kristallwachstumszeit einen größeren Überschuß an Fällungsmittel bis zu etwa der doppelten stöchiometrisch erforderlichen Menge. Hierbei kann es von Vorteil sein, wenn man die Temperatur nochmals kurz auf etwa 110°C erhöht und wieder auf etwa 95°C abfallen läßt und erst dann weiteres Fällungsmittel in einem etwa 50 bis 100%igen Überschuß zugibt.

Wenn alles Fällungsmittel zugegeben ist, läßt man auf etwa Zimmertemperatur abkühlen und isoliert und wäscht dann das Fällungsprodukt in üblicher Weise. Als Waschlösung kann eine wäßrige Lösung eines Alkalimetallchlorids Verwendung finden, zweckmäßig jedoch auch hier im Hinblick auf die Aufarbeitung der Waschwässer eine wäßrige Salzsäure. In diesem Falle kann ein Teil des salzsauren Waschfiltrats bei einer nachfolgenden Kristallisation wieder eingesetzt werden.

Man erhält so ein Dihydrochlorid mit einem Wassergehalt von etwa 4 bis 8%, einem Sulfatgehalt von höchstens 0,1% und einem Diazowert, bezogen auf freie Base, von 77,6% (was dem theoretischen Wert entspricht). Diese Reinheit entspricht den Anforderungen für die Herstellung von Farbmitteln.

Die vereinigten Filtrate bilden eine etwa 32%ige schwefelsaure Mutterlauge, die etwa 3% Salzsäure und etwa 0,6% organischen Kohlenstoff enthält. Aus dieser Mutterlauge kann durch einfache Destillation eine wäßrige Salzsäure zurückerhalten werden, während die verbleibende Schwefelsäure direkt der üblichen Regeneration zugeführt werden kann.

Bei den vorstehenden Angaben wie auch im folgenden Beispiel beziehen sich Prozentangaben auf das Gewicht.

## Beispiel

### Umlagerung

In einem Rührkolben werden 427 g (1,68 Mol) 2,2'-Dichlor-hydrazobenzol in Form von 1780 g einer 24%igen xylolischen Lösung vorgelegt und bei 25 bis 35°C innerhalb von 2 Stunden unter Rühren mit 1710 g einer 62,5%igen Schwefelsäure versetzt. Nach 1stündigem Nachrühren bei 40°C ist die Umlagerung beendet. Nach Zusatz von 900 g Wasser wird die Reaktionsmischung auf 95 bis 100°C erhitzt. Nach Abschalten des Rührers trennt sich der Ansatz in zwei sich scharf abhebende Phasen auf. Die Xylolphase wird abgetrennt und durch Destillation regeneriert.

### Herstellung des Dihydrochlorids

Die schwefelsaure Phase wird nach Klärung mit 20 g Aktivkohle zunächst mit 230 g 30%iger Salzsäure versetzt und bis auf 110°C erhitzt (bei dieser Temperatur tritt ein leichter Rückfluß auf). Hierbei ist darauf zu achten, daß alle Ausfällungen bis etwa 107/108°C in Lösung gegangen sind. Unter langsamem Rühren läßt man die Temperatur innerhalb von 2 Stunden auf etwa 95°C abfallen. Hierbei beginnt die Kristallisation des Hydrochlorids ab etwa 107°C; größere Kristalle bilden sich dann zwischen 100 und 95°C. Zur Vervollständigung der Kristallisation läßt man in den folgenden 3 Stunden bei einer Temperatur von etwa 95 bis 98°C gleichmäßig weitere 150 g 30%ige Salzsäure zulaufen. Nachdem man innerhalb von weiteren zwei Stunden nochmals 378 g 30%ige Salzsäure zugeführt hat, läßt man unter Rühren auf 20 bis 25°C abkühlen und trennt die Kristallsuspension über eine Schälzentrifuge. Das Dihydrochlorid wird 6mal mit 200 g 30%iger Salzsäure gewaschen. Der Rückstand enthält 4 bis 8% Wasser. Nach Trocknen erhält man 470 g (85,8% der Theorie, bezogen auf eingesetztes 2,2'-Dichlor-hydrazobenzol) 3,3'-Dichlor-4,4'-diamino-biphenyl-dihydrochlorid mit einem Sulfatgehalt von höchstens 0,1% und einem Diazotierungswert von 77,6%, der dem theoretischen Wert für die freie Base entspricht.

### Aufarbeitung der Mutterlauge

758 g des Waschfiltrats werden für eine folgende Kristallisation eingesetzt. Aus der vereinigten schwefelsauren Mutterlauge (32,4% $H_2SO_4$, 3% HCl, 0,61% org. C) und dem restlichen salzsauren Waschfiltrat wird durch Destillation eine wäßrige Salzsäure zum Aufkonzentrieren erhalten, während im Sumpf eine Schwefelsäure (1,64% org. C) anfällt, die nach dem Pauling-Verfahren aufkonzentriert werden kann.

## Patentansprüche

1. Verfahren zur Herstellung von 3,3'-Dichlor-4,4'-diamino-biphenyl-dihydrochlorid aus der schwefelsauren wäßrigen Lösung des Sulfats durch Fällen mit einem Chloridionen abgebenden Mittel, Isolieren und Waschen des Fällungsprodukts mit einer wäßrigen, ein Chloridionen abgebendes Mittel enthaltenden Lösung, dadurch gekennzeichnet, daß man die annähernd gesättigte wäßrige Lösung des Sulfats auf eine Temperatur im Bereich von über 100°C bis zur Rückflußtemperatur erhitzt, hierbei etwa 50 bis etwa 70% der stöchiometrisch erforderlichen Menge des Fällungsmittels zusetzt, auf eine Temperatur im Bereich von etwa 90 bis unter 100°C abkühlt, weiteres Fällungsmittel zugibt und auf etwa Zimmertemperatur abkühlt.

2. Verfahren nach Anspruch 1, dadurch gekennzeichnet, daß das Chloridionen abgebende Fällungsmittel Salzsäure ist.

3. Verfahren nach Anspruch 1 und 2, dadurch gekennzeichnet, daß man im Temperaturbereich von über 100°C bis zur Rückflußtemperatur etwa 65% der stöchiometrisch erforderlichen Menge des Fällungsmittels und im Bereich von etwa 90 bis unter 100°C weiteres Fällungsmittel bis zu etwa dem Doppelten der stöchiometrisch erforderlichen Menge zusetzt.

4. Verfahren nach Anspruch 1 bis 3, dadurch gekennzeichnet, daß man im Temperaturbereich von etwa 90 bis unter 100°C zunächst die restliche, stöchiometrisch erforderliche Menge an Fällungsmittel oder einen geringen Überschuß zusetzt und erst zu einem späteren Zeitpunkt einen größeren Überschuß an Fällungsmittel zugibt.

5. Verfahren nach Anspruch 1 bis 4, dadurch gekennzeichnet, daß man das ausgefallene Dihydrochlorid mit wäßriger Salzsäure wäscht.

6. Verfahren nach Anspruch 5, dadurch gekennzeichnet, daß das Waschfiltrat für eine nachfolgende Kristallisation eingesetzt wird.

## Claims

1. Process for the manufacture of 3,3'-dichloro4,4'-diamino-biphenyl-dihydrochloride from the aqueous, sulfuric-acid containing solution of the sulfate by precipitation with a chloride-ion-yielding agent, isolation and washing of the precipitated product with an aqueous solution containing a chloride-ion-yielding agent, characterized in heating the nearly saturated aqueous solution of the sulfate to a temperature in the range of from above 100°C to reflux temperatur while adding about 50 to about 70% of the stoichiometrically required amount of the precipitating agent, then cooling it to a temperature in the range of from about 90 to below 100°C, adding further precipitating agent, and cooling to about room temperature.

2. A process according to claim 1, wherein the chloride-ion-yielding precipitating agent is hydrochloric acid.

3. A process according to claim 1 or 2, characterized in adding, in the temperature range of from above 100°C to reflux temperature, about 65% of the stoichiometrically required amount of the precipitating agent and further precipitating agent up to about twice the stoichiometrically required amount in the temperature range of from about 90°C to below 100°C.

4. A process according to claim 1 to 3, characterized in adding first, in the temperature range of from about 90°C to below 100°C, the remaining stoichiometrically required amount of the precipitating agent or a slight excess thereof, and a larger excess of the precipitating agent at a later moment.

5. A process according to claim 1 to 4, characterized in washing the precipitated dihydrochloride with aqueous hydrochloric acid.

6. A process according to claim 5, characterized in reusing the washing filtrate for a subsequent crystallization.


**Revendications**

1. Procédé pour préparer le dichlorhydrate du dichloro-3,3′ diamino-4,4′ biphényle à partir d'une solution aqueuse sulfurique du sulfate, par précipitation au moyen d'un agent cédant des ions chlorures, isolement et lavage du produit précipité au moyen d'une solution aqueuse contenant un agent cédant des ions chlorures, procédé caractérisé en ce qu'on chauffe à une température supérieure à 100°C et pouvant aller jusqu'à la température du reflux la solution aqueuse, à peu près saturée, du sulfate, on ajoute alors d'environ 50 à environ 70% de la quantité stochiométrique de l'agent de précipitation, on refroidit à une température inférieure à 100°C mais au moins égale à environ 90°C, on ajoute une quantité supplémentaire de l'agent de précipitation et on refroidit à peu près à la température ambiante.

2. Procédé selon la revendication 1, caractérisé en ce que l'agent de précipitation cédant des ions chlorures est l'acide chlorhydrique.

3. Procédé selon l'une des revendications 1 et 2, caractérisé en ce que la quantité de l'agent de précipitation que l'on ajoute à une température supérieure à 100°C et pouvant aller jusqu'à la température du reflux représente environ 65% de la quantité stoechiométrique, et la quantité supplémentaire de l'agent de précipitation que l'on ajoute à une température au moins égale à environ 90°C et inférieure à 100°C peut aller jusqu'à environ le double de la quantité stochiométrique.

4. Procédé selon l'une quelconque des revendications 1 à 3, caractérisé en ce qu'on ajoute, à une température au moins égale à environ 90°C et inférieure à 100°C, d'abord la quantité stoechiométrique restante de l'agent de précipitation ou un léger excès, et on ajoute, mais seulement à un moment ultérieur, un plus grand excès de l'agent de précipitation.

5. Procédé selon l'une quelconque des revendications 1 à 4, caractérisé en ce qu'on lave avec de l'acide chlorhydrique aqueux le dichlorhydrate qui a précipité.

6. Procédé selon la revendication 5, caractérisé en ce qu'on utilise le filtrat de lavage pour une cristallisation ultérieure.